# EUROPEAN PATENT APPLICATION

(11) **EP 4 826 371 A2**
(43) Date of publication of application: **21.10.2026**
(21) Application number: 26168969.9
(22) Date of filing: 30.03.2026
(51) Int. Cl.: G01N 27/407

(54) **PROTON CONDUCTOR GAS SENSOR**

(30) Priority: 15.04.2025 JP 2025066694
(71) Applicant: Figaro Engineering Inc., Mino, Osaka 562-8505 (JP)
(72) Inventor: SUGITA, Norio, Mino, Osaka, 562-8505 (JP); FUJITANI, Shono, Mino, Osaka, 562-8505 (JP); TANAKA, Yuya, Mino, Osaka, 562-8505 (JP)
(74) Representative: Kuhnen & Wacker Patent- und Rechtsanwaltsbüro PartG mbB

(57) **Abstract**

A proton conductor gas sensor has an MEA (14) which has a proton conductor membrane (15), positioned over the bottom portion (5) of a metal can (4), and a sealing cap (9) over the MEA (14). The space between the side portion (7) of the metal can (4) and the sealing cap (9) is sealed by a ring-shaped gasket (22) made of synthetic resin. A ring-shaped synthetic rubber member (26,30), more easily deformable than the gasket (22) is positioned between the bottom portion (5) of the gasket (22) and the bottom portion (5) of the metal can (4).

## Description

### Technical Field

The present invention relates to a proton conductor gas sensor, in particular, to improvements in the impact resistance of the gas sensor.

### Background Art

Patent Document 1 (JP2011-220905A) discloses a proton conductor gas sensor. The gas sensor uses a membrane electrode assembly (MEA) which comprises a proton conductor membrane, a pair of electrodes (a detection electrode and a counter electrode), and a pair of gas diffusion membranes. The MEA is housed in the bottom portion of a metal can. A metal sealing cap containing a filter, such as activated carbon, is placed over the MEA. A ring-shaped gasket is positioned between the sealing cap and the metal can such that the gasket is compressed when the metal can is caulked. This configuration results in the following functions:
* The pressure from the gasket positions correctly the sealing cap and the MEA.
* The sealing cap and the metal can are mutually insulated.
* The gap between the sealing cap and the metal can is air-tightly sealed. By the way, Patent Document 1 proposes a polyolefin-based elastomer as a material for the gasket.

In the gas sensor according to Patent Document 1, the metal can is connected to the counter electrode of the MEA, and the sealing cap is connected to the detection electrode of the MEA. The current between the two electrodes is proportional to the amount of gas that reached the MEA and was decomposed at the detection electrode. Therefore, a sensor signal proportional to the gas concentration is obtained. Such gas sensors have been used for the detection of CO, H2, ethanol, acetone, and the like.

### Prior Art Documents List

### Patent Documents

Patent Document 1: JP2011-220905A

### Summary of the Invention

### Problem to be Solved by the Invention

The present inventors tested gas sensors similar to those in Patent Document 1 to impact resistance tests. As a result, the gas sensitivity of the gas sensors increased. The following mechanism is considered to cause the increase in gas sensitivity. The impact applied during the test made the seal between the gasket and the metal can imperfect. Consequently, a gas flow path was generated from the outside to the detection electrode through the gap between the metal can and the gasket, and therefore, the gas sensitivity increased.

The object of this invention is to improve the impact resistance of proton conductor gas sensors.

### Means for Solving the Problem

The proton conductor gas sensor according to the present invention comprises:
a metal can having a flat bottom portion and a ring-shaped side portion;
an MEA (membrane electrode assembly) having a proton conductor membrane and positioned over the bottom portion of the metal can;
a sealing cap made of metal, having an internal space accommodating a filter, a first opening introducing outside air into the internal space, and a second opening supplying the outside air processed by the filter to the MEA, positioned on the opposite side of the bottom portion of the metal can from the MEA; and
a gasket having a ring shape and positioned between the side portion of the metal can and the sealing cap, wherein an edge of the side portion of the metal can on the opposite side of the bottom portion of the metal can is bent towards the sealing cap and presses the gasket.

The gasket is made of synthetic resin. A synthetic rubber member having a ring shape, more easily deformable than the gasket by pressure, is airtightly positioned between a bottom portion of the gasket and the bottom portion of the metal can and surrounds the MEA.

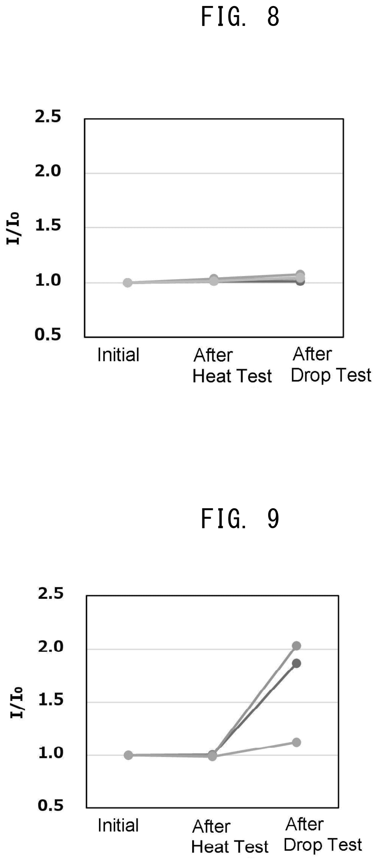

Applying an impact to proton conductor gas sensors increased often their gas sensitivity (see Fig. 9, for a conventional proton conductor gas sensor). In Fig. 9, the increase in the gas sensitivity was approximately twice at maximum. This suggests that the impact loosened the seal between the gasket and the bottom portion of the metal can and generated a gas flow path through an interface between the gasket and metal can. It is further suggested that the outside air, introduced to the vicinity of the MEA through the gas flow path, diffused into the MEA from the upper portion of the MEA and increased the gas sensitivity.

According to the present invention, a synthetic rubber member, having a ring shape, more easily deformable than the gasket by pressure, is airtightly positioned between a bottom portion of the gasket and the bottom portion of the metal can and surrounds the MEA. Then, the gap between the rubber member and the bottom portion of the metal can is constantly airtightly sealed, and the unneeded diffusion of outside air into the MEA is prevented (see Fig. 8 for an embodiment of proton conductor gas sensor). As a result, the proton conductor gas sensor is improved in its impact resistance according to the invention. Since typical impact is drop impact, the increase in the gas sensitivity by a drop impact test is reduced by the synthetic rubber member.

Preferably, a bottom plate having a circular disk-like shape, made of metal, and having a thickness larger than that of the bottom portion of the metal can, is positioned on the bottom portion of the metal can, and the MEA and the synthetic rubber member are positioned on the bottom plate. Since the bottom plate is less prone to deformation due to impact compared to the bottom portion of the metal can, gaps are less likely to occur between the synthetic rubber member and the bottom plate.

Preferred materials for the gasket include high-density polyethylene, nylon, and PTFE (polytetrafluoro-ethylene). Preferred materials for the synthetic rubber member include NBR rubber (acrylonitrile-butadiene rubber), silicone rubber, FKM, PTFE rubber (polytetrafluoro-ethylene rubber), or nitrile rubber. FKM is a fluoro-elastomer sold under the trade names such as Viton (Viton is a registered trademark of Chemours Company). PTFE rubber has lower hardness than PTFE used for gaskets and is sold under the trade names such as Teflon rubber (TEFLON is a trademark of DuPont.)

If the gasket were made of the same material as the synthetic rubber member, when the metal can is caulked, insufficient pressure would be applied from the gasket to the sealing cap and MEA, and their accurate positioning is prevented. Consequently, the variation in gas sensitivity increases, and the gas sensitivity becomes to more fluctuate due to impact or the like.

The synthetic rubber member is ring-shaped, and its precise shape is arbitrary. Furthermore, the member is not limited to sheet form. However, when the synthetic rubber member is an O-ring made of synthetic rubber, the synthetic rubber member is easily produced or purchased.

Proton conductor gas sensors, having a diffusion control plate with a diffusion control hole, for supplying outside air supplied from a second opening of the sealing cap to the MEA, have been known. The diffusion control plate is disposed between the MEA and the sealing cap, and the diffusion control hole has a smaller diameter than the second opening. Since it is easier to form an opening of precise size in the diffusion control plate than in the sealing cap, the diffusion control plate with the diffusion control hole enables more accurate control of the outside air supply to the MEA.

In this type of proton conductor gas sensors, the diffusion control plate is pressed towards the MEA near the diffusion control hole. Consequently, MEA materials intrude sometimes into the diffusion control hole, and the air permeability of the hole is changed. This leads to the variation in the gas sensitivity. Therefore, it is preferable to provide a protrusion on the diffusion control plate around the diffusion control hole, protruding towards the opposite side (the filter side) from the MEA and to create a gap between the interior of the protrusion and the MEA. This prevents MEA materials from entering into the diffusion control hole, keeps a constant supply of outside air to the MEA, and reduces the variations in gas sensitivity.

The gas flow path between the gasket and the inner circumference of the metal can is sealed by the ring-shaped synthetic rubber member. The inventors have further investigated whether another unintended gas flow paths were present. As a result, the inventors have found a minute gas flow path between the bottom surface of the sealing cap and the top surface of the diffusion control plate, and the gas flow path allowed gas to move from the opening of the sealing cap to the MEA. Since both the sealing cap and the diffusion control plate are made of metal and have fine surface unevenness, the unneeded gas flow path was present. The gas moved to the edge of the diffusion control plate reached the MEA through a gap between the MEA and the synthetic rubber member.

To shut off this gas flow path, the outer circumference of the diffusion control plate is preferably extended to a position between the gasket and the synthetic rubber member. As a result, the gap between the MEA and the synthetic rubber is covered by the diffusion control plate, and the gas flow path is shut off.

When gas moves along the top surface of the diffusion control plate to its outer circumference, the interface between the synthetic rubber member and the metal can, or the interface between the diffusion control plate and the synthetic rubber member, could become a further gas flow path. However, since the synthetic rubber member is compressed by the pressure due to the caulking, tightly adheres to the bottom portion of the metal can and also to the bottom portion of the diffusion control plate, it is difficult for gas to pass through these interfaces. Preferably, the outer circumference of the diffusion control plate is entirely positioned between the gasket and the synthetic rubber member.

Instead of extending the diffusion control plate towards the inner circumference of the metal can, an airtight and electrically conductive material film may be provided between the diffusion control plate and the bottom surface of the sealing cap. This film seals the interface between the diffusion control plate and the sealing cap and electrically connects the diffusion control plate to the sealing cap with its electrical conductivity.

### Brief Description of the Drawings

- Fig. 1:: A cross-sectional view of a proton conductor gas sensor according to a basic embodiment.
- Fig. 2:: An enlarged cross-sectional view of the main part of the proton conductor gas sensor of Fig. 1.
- Fig. 3:: A plan view of the gasket in the basic embodiment.
- Fig. 4:: A plan view of the O-ring in the basic embodiment.
- Fig. 5:: A cross-sectional view of the O-ring in the basic embodiment.
- Fig. 6:: A cross-sectional view of the O-ring in a modified embodiment.
- Fig. 7:: A flowchart indicating the flow of the durability test including the drop impact test in the embodiment.
- Fig. 8:: A characteristic view indicating the results of the durability test regarding the gas sensors according to the basic embodiment.
- Fig. 9:: A characteristic view indicating the results of the durability test regarding a conventional gas sensors.
- Fig. 10:: A cross-sectional view of the proton conductor gas sensor according to a second embodiment.
- Fig. 11:: An enlarged cross-sectional view of the main part of the diffusion control plate and the MEA according to the second embodiment.
- Fig. 12:: An enlarged cross-sectional view of the main part of the diffusion control plate and the MEA according to the basic embodiment.
- Fig.13:: A characteristic view indicating the distributions of initial CO sensitivities according to the second embodiment and the basic embodiment.
- Fig. 14:: A cross-sectional view of the proton conductor gas sensor according to the best embodiment.
- Fig. 15:: A cross-sectional view of the gas sensor according to the best embodiment, in a plane parallel to the bottom plate.
- Fig.16:: A cross-sectional view of a modified embodiment with another diffusion control plate modified from one in the best embodiment.
- Fig.17:: A cross-sectional view of a second modified embodiment with another diffusion control plate modified from the best embodiment.
- Fig. 18:: A cross-sectional view of a third modified embodiment with another diffusion control plate modified from the best embodiment.
- Fig. 19:: A characteristic view indicating the distribution of CO sensitivity after drop test, according to the second embodiment.
- Fig. 20:: A characteristic view indicating the distribution of CO sensitivity after drop test according, to the best embodiment.

### Features for Carrying out the Invention

The following embodiments illustrate the implementation of the present invention.

### Embodiments

### Structure of the Gas Sensor

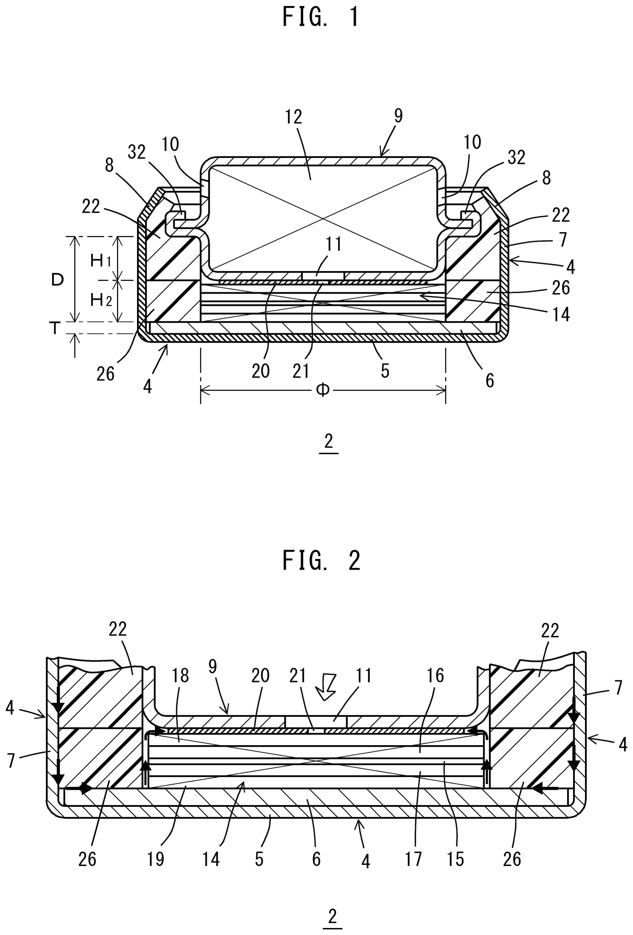

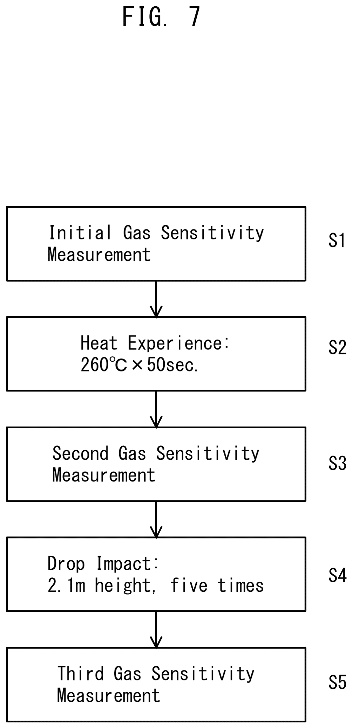

Figs. 1 to 8 show a basic embodiment. In the drawings, indicated by 2 is a proton conductor gas sensor comprising a metal can 4 that is circular in plan view, with a flat bottom portion 5, and a metal bottom plate 6 placed on the bottom portion. The thickness of the bottom plate 6 is greater than that of the bottom portion 5, and the bottom plate 6 has higher rigidity than the bottom portion 5. Indicated by 7 is the side peripheral of the metal can 4, circular in plan view, and 8 is the caulked part on the upper side of the side peripheral 7.

Indicated by 9 is a sealing cap formed by welding two circular plate-shaped metal members, with a filter 12 housed within its internal space to adsorb or reactively remove unneeded gases. Indicated by 10 is a first opening for introducing outside air, provided at the top or side surface of the sealing cap 9. Indicated by 11 is a second opening, provided at the bottom portion of the sealing cap 9, for supplying outside air that has passed through the filter 12 to the MEA 14 via a diffusion control plate 20. The diffusion control plate 20 is a thin metal plate that supplies outside air to the MEA 14 through diffusion control holes 21.

The MEA 14 includes a proton conductor membrane 15 at its center in the thickness direction. As shown in the upper and lower parts of Figs. 1 and 2, a detection electrode 16 is provided over the membrane 15, and a counter electrode 17 is provided under it. A gas diffusion membrane 18, made of materials such as activated carbon or carbon sheet, is provided over the detection electrode 16, and another gas diffusion membrane 19 made of a similar material is provided under the counter electrode 17.

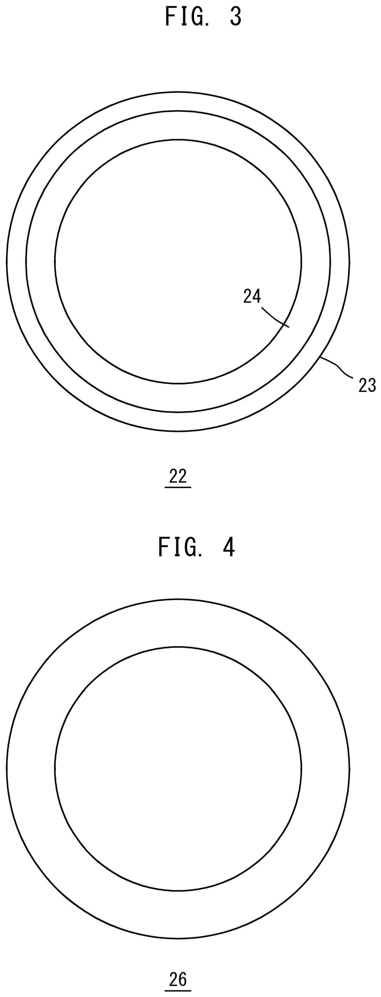

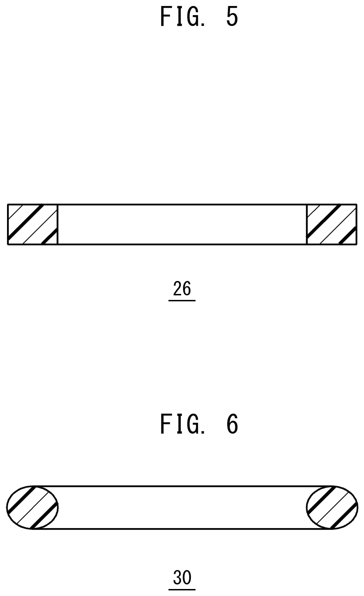

Indicated by 22 is a gasket, and 26 is an O-ring. The O-ring 26 is positioned such that it substantially surrounds the MEA 14, while the gasket 22 is positioned to substantially surround the sealing cap 9. The gasket 22 is shown in plan view in Fig. 3, having a circular protrusion 23 on its upper outer periphery and a circular base 24. The O-ring 26 is circular in plan view, as shown in Fig. 4, and has a rectangular cross-sectional shape, as shown in Fig. 5. However, the cross sectional shape may be deviated from circular, such as elliptical to circular, as shown in the O-ring 30 of Fig. 6.

When the metal can 4 is caulked at the caulked part 8, the gasket 22 is compressed, and then, presses the sealing cap 9 relative to the MEA 14. Furthermore, due to the pressure generated during caulking, the O-rings 26 and 30 are compressed and fill the gap between the bottom plate 6, preventing gas passage between the O-rings 26, 30 and the bottom plate 6. Therefore, the O-rings 26, 30 are softer than the gasket 22. The O-rings 26, 30 may be replaced by a ring-shaped sheet or an annular sheet with a ring-like protrusion extending towards the bottom plate 6. A ring-like layer of soft rubber, between the gasket 22 and the bottom plate 6, is also usable. The material of the gasket may be divided into upper and lower parts, with the combined size being that of gasket 22 and O-ring 26. A soft rubber member may be used instead of the O-ring 26. In this specification, an elastomer is considered a type of rubber. Furthermore, the ring-shaped member need not be physically independent from the gasket.

The material of the gasket 22 is synthetic resin, preferably nylon 66, fluororesin such as PTFE, or high-density polyethylene. In the embodiment, gasket 22 is made of high-density polyethylene. The material of O-rings 26 and 30 is preferably NBR rubber, silicone rubber, FKM, PTFE rubber, or nitrile rubber.

The gasket 22 preferably has a Shore D hardness of 50 or more and 90 or less, more preferably has a Shore D hardness of 55 or more and 90 or less, and particularly preferably has a Shore D hardness of 60 or more and 90 or less. The O-rings 26 and 30 preferably have a Shore A hardness of 50 or more and 90 or less. More preferably, the Shore A hardness is 50 or more and 85 or less. Particularly preferably, the Shore A hardness is 50 or higher and 80 or lower. While the material and hardness are specified here for O-rings 26 and 30, the same applies to synthetic rubber member of shapes other than O-rings. Shore D hardness is a unit indicating hardness for plastics and similar materials, and Shore A hardness is a unit indicating hardness for rubber and similar materials. Furthermore, a Shore D hardness of 40 is approximately equivalent to a Shore A hardness of 90. Shore hardness is measured using a commercially available Shore hardness tester conforming to ASTM D2240.

Examples of the sizes of the gasket 22, the O-ring 26, etc. are described. In the embodiment, the distance D from the bottom portion of the flange 32 protruding from the side of the sealing cap 9 to the bottom plate 6 is 2 mm. Within this, the height H1 occupied by the gasket 22 is, for example, 0.8 to 1.5 mm, and the height H2 occupied by the O-ring 26 is 1.2 to 0.5 mm. Furthermore, the gasket 22 and the O-ring 26 have an outer diameter of, for example 13 mm, and an inner diameter of, for example, 11 mm. The thickness T of the bottom plate 6 is 0.5 to 0.7 mm, and the thickness of the metal can 4 is 0.2 mm.

When assembling the gas sensor 2, the base plate 6 is placed on the bottom portion 5 of the metal can 4, and then the MEA 14 is placed on the base plate 6. Then, the O-ring 26 and gasket 22 are placed around the MEA 14. Next, the sealing cap 9 is placed inside the gasket 22 over the MEA 14, and the caulked part 8 of the metal can 4 is caulked.

### Test of Gas Sensor

Fig. 5 shows the evaluation procedure of the characteristics regarding the gas sensors 2. The initial gas sensitivities (e.g., initial CO sensitivities) were measured after manufacturing (Step 1). Then, for a heat resistance test, the sensors were placed in a chamber at 260°C for 50 seconds (Step 2). After the heating, the gas sensitivities were measured (Step 3). As a drop impact test, the sensors were dropped 5 times from a height of 2.1 m onto a concrete floor (Step 4), and then, the gas sensitivities were measured (Step 5).

A high-density polyethylene gasket (Shore D hardness: 70) was used as the material of the gasket 22, and, as the O-ring 26, NBR rubber (Shore A hardness: 70) was used. In the test, four gas sensors 2 according to the embodiment were tested (Fig. 8). Three conventional gas sensors without the O-ring 26 were tested (Fig. 9). In the conventional gas sensors, the space for the O-ring 26 was occupied by the gasket 22.

All the gas sensors exhibited sufficient heat resistance. However, the conventional sensors showed a maximum twofold increase in gas sensitivities after the drop test. In the embodiment gas sensors, the increase in gas sensitivities were less than 10% which were significantly smaller than those of the conventional gas sensors.

### Second Embodiment

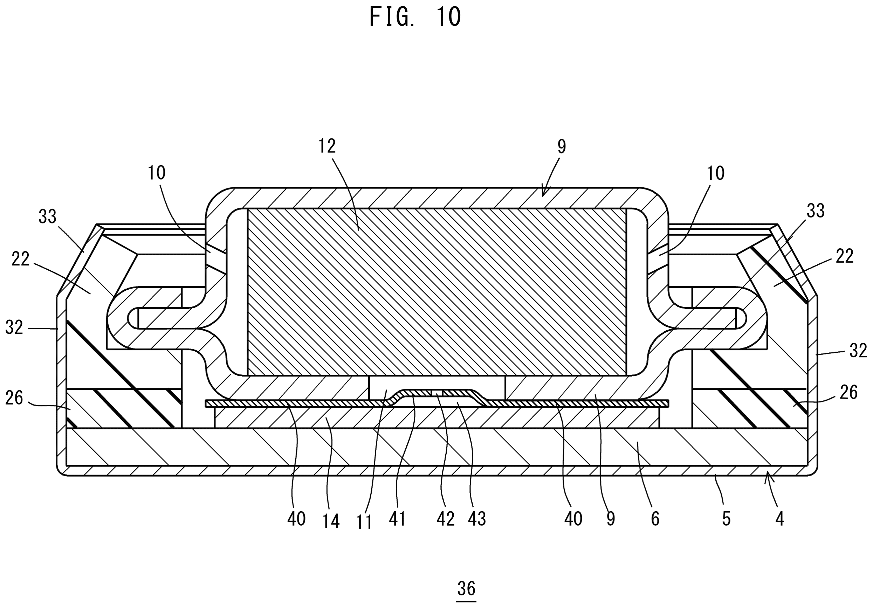

Figs. 10 to 13 show a second embodiment that reduces sensitivity variation which is caused by MEA 14 material entering the diffusion control hole 21. Unless specifically noted, it is similar to the basic embodiment shown in Figs. 1 to 8. Specifically, the gasket 22 and the O-ring 26 are configured similarly to the basic embodiment in Figs. 1 to 8. Alternatively, an O-ring 30 may be used instead of the O-ring 26.

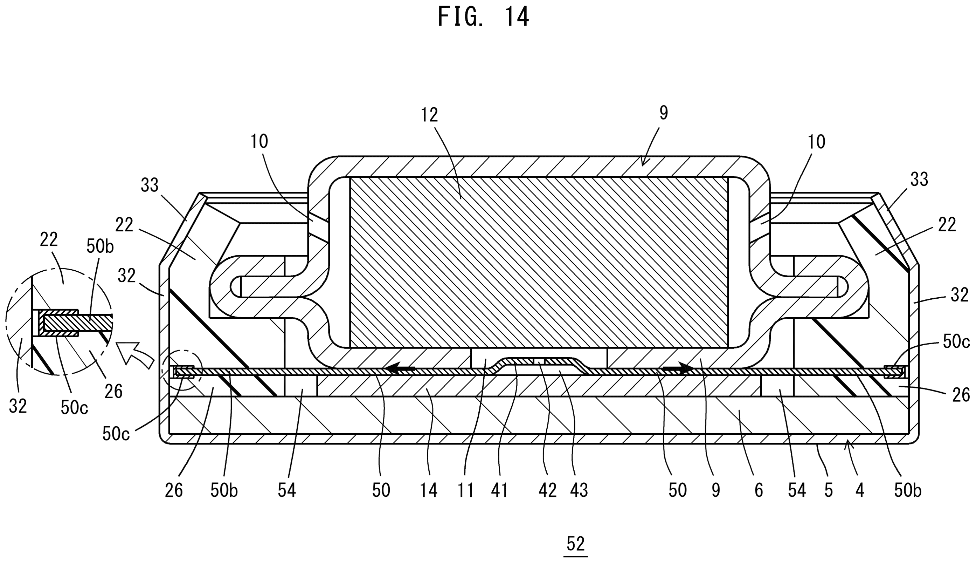

The MEA 14 receives pressure from the gasket 22 via the sealing cap 9, and therefore, the materials of the MEA 14 may enter the diffusion control hole 21 in Fig. 1. This causes variation in gas sensitivity. Therefore, the gas sensor 36 according to the second embodiment has a protrusion 41 at the center of the diffusion control plate 40 and within the opening 11. The protrusion 41 has a diffusion control hole 42 at its center. As a result, a gap 43 between the MEA and the protrusion 41 prevents the MEA material from entering the diffusion control hole 42. In Fig. 14, denoted by 46 is an assembly of a proton conductor membrane and a pair of electrodes.

Examples of dimensions regarding the diffusion control plate 40 are described The diameter of the opening 11 is, for example, 2.5 mm, the outer diameter *ϕ* of the protrusion 41 is, for example, 1.5 mm, the diameter d of the diffusion control hole 42 is, for example, 0.1 mm, the height b by which the protrusion 22 is lifted is, for example, 0.2 mm, and the thickness t of the diffusion control plate 40 is, for example, 0.1 mm.

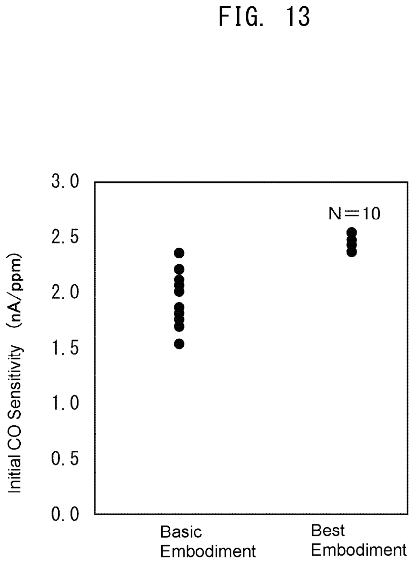

Fig. 13 shows the advantageous effect according to the second embodiment. The basic embodiment in the drawing is one shown in Figs. 1 to 8. It had a greater variation in initial gas sensitivities compared to the second embodiment. The second embodiment used the O-ring 26 to prevent uncontrolled flow of outside air, similarly to the basic embodiment in Figs. 1 to 8.

### Best Embodiment

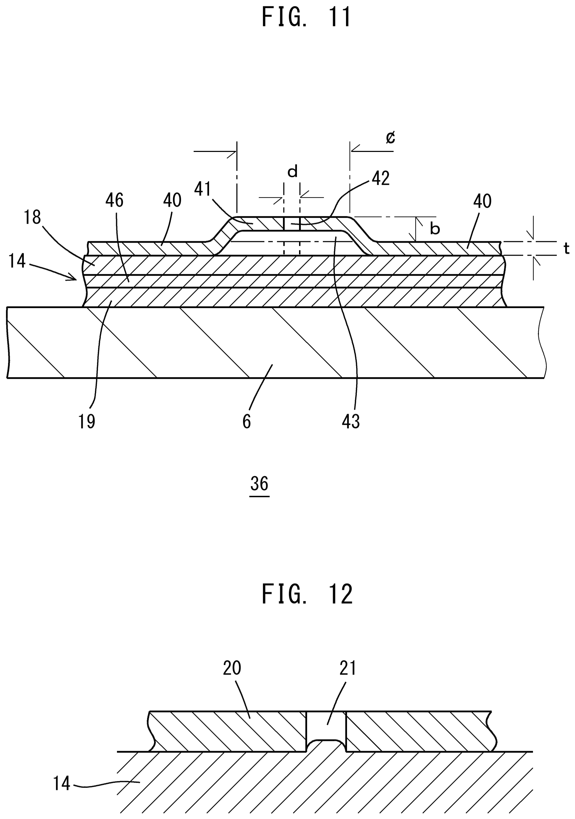

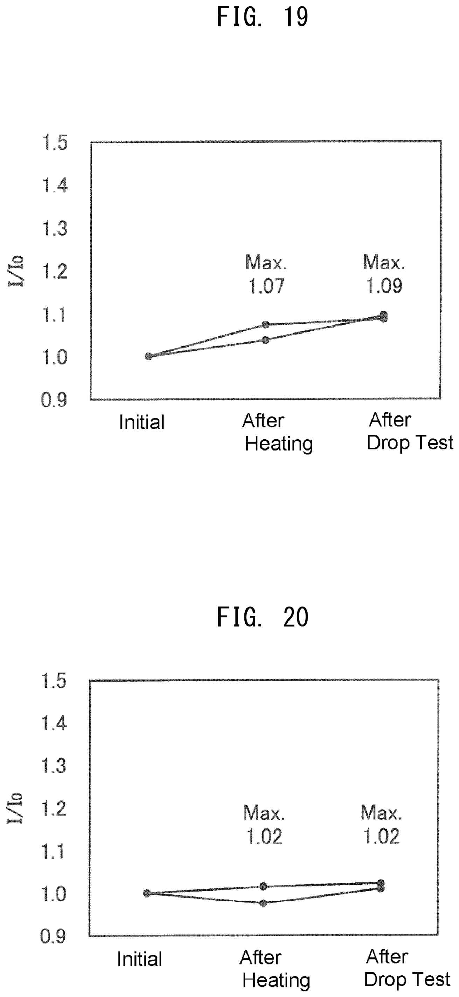

Figs. 14 to 18 show the best embodiment and its modifications. Fig. 20 shows the CO sensitivity variations after the drop test according to the best embodiment, and Fig. 19 shows the variations according to the second embodiment after the same test. The best embodiment was the same as the second embodiment in Figs. 10 and 11, except for points specifically pointed out. According to the best embodiment and its modifications, the protrusion 41 was present at the center of the diffusion control plate, and the diffusion control hole 42 was provided on its top surface. However, as in the basic embodiment of Figs. 1 to 8, the protrusion 41 may be omitted. Furthermore, unless otherwise noted, the descriptions concerning the basic embodiment of Figs. 1 to 8 apply to the best embodiment and its modifications. For example, instead of the square O-ring 26 of Fig. 5, the O-ring 30 of Fig. 6 may be used.

In the embodiments, the flow path between the gasket 22 and the metal can 4 is sealed by the O-ring 26. The inventors have discovered that another gas flow path may be present between the bottom surface of the sealing cap 9 and the top surface of the diffusion control plate 40, as indicated by the arrow in Fig. 14. Both the sealing cap 9 and the diffusion control plate 40 are made of metal and have fine unevenness on their surfaces; these unevenness forms the gas flow path. In the gas sensors of Figs. 1 and 10, gas flowing from the opening 11 of the sealing cap 9 through the interface between the sealing cap 9 and the diffusion control plate 40 reaches the MEA 14 through the gap 54 shown in Fig. 14, causing variation in the sensor's gas sensitivity. The existence of such gas flow paths is inferred from Figs. 19 and 20.

To eliminate the effect of this gas flow path, the diffusion control plate 40 is extended to an area over the O-ring 26. This prevents gas diffusion through the gap between the O-ring 26 and the MEA 14 (a portion indicated by reference numeral 54 in Fig. 14), as shown in Fig. 10. According to the best embodiment, the interface between the sealing cap 9 and the diffusion control plate 40 does not need to be made airtight.

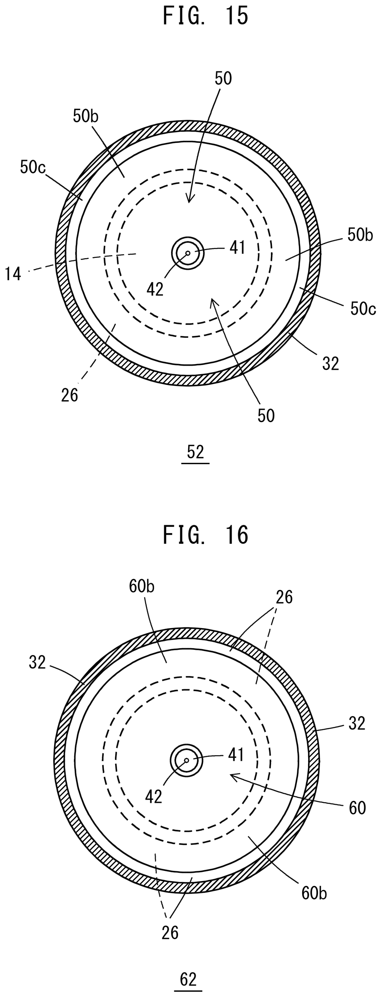

Figs. 14 and 15 show the structure of the proton conductor gas sensor 52 and the diffusion control plate 50 according to the best embodiment. The diffusion control plate 50 is circular, with its entire outer periphery either contacting the flange 32 of the metal can or being located in the vicinity of the flange 32. The outer periphery of the diffusion control plate 50 extends at least to the upper part of the O-ring 26, as viewed from the diffusion control hole 42, covering the gap 54. Furthermore, the caulking is performed such that the thickness of the O-ring 26 after the caulking is made substantially equal to the thickness of the MEA 14. The portion of the diffusion control plate 50 located over the O-ring 26 is referred to as the extended part 50b. In Figs. 14 and 15, since the metal diffusion control plate 50 extends at least in the vicinity of the flange 32, an insulating film 50c is provided on the outer circumference of the diffusion control plate 50, in particular on the outer circumference of the extended part 50b. The left side of Fig. 14 shows the insulating film 50c and its surroundings. If the extended part 50b is in contact with the flange 32, the insulating film 50c prevents the shortcircuiting between the detection electrode and the counter electrode of the MEA 14.

If gas passing through the gap between the diffusion control plate 50 and the bottom surface of the sealing cap 9 reaches the flange 32, it will not pass through the gap between the O-ring 26 and the bottom plate 6. Therefore, the gas does not reach the MEA 14, it cannot pass the interface between the O-ring 26 and the bottom plate 6. Therefore, the gas does not reach the MEA 14. Furthermore, the space between the gasket 22 and the extended part 50b of the diffusion control plate 50 is also substantially airtight. Therefore, the influence of the flow path between the diffusion control plate 50 and the bottom surface of the sealing cap 9 is made very small.

Fig. 16 shows a modified embodiment where the outer circumference of the extended part is restricted to a position over the O-ring 26, closer than the flange 32. Denoted by 62 is another proton conductor gas sensor, 60 is the diffusion control plate, and 60b is the extended part, which does not contact the flange 32.

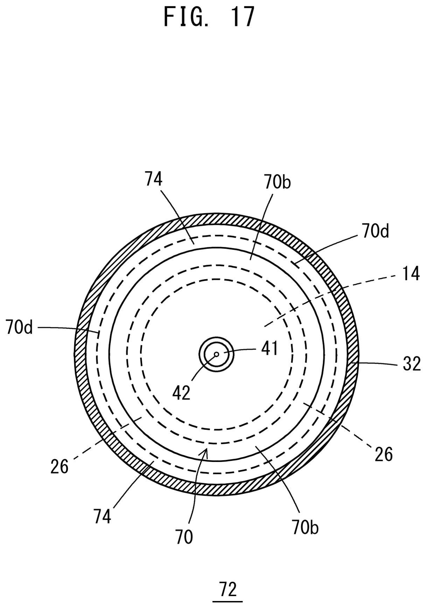

Fig. 17 shows a second modified embodiment where an insulating film 74 is provided instead of the insulating film 50c shown in Figs. 14 and 15, indicated by 72 is the proton conductor gas sensor, 70 is the diffusion control plate, and 70b is its extended part. The ring-shaped insulating film 74 is fixed to the outer circumference 70d of the extended part 72 by adhesion or thermal bonding, etc. The outer circumference 70d of the extended part 70b is positioned so as not to contact the inner circumference of the flange 32.

The gas sensors 62 and 72 shown in Figs. 16 and 17 also prevent gas flowing between the diffusion control plates 60 and 70 and the sealing cap 9 from reaching the MEA 14.

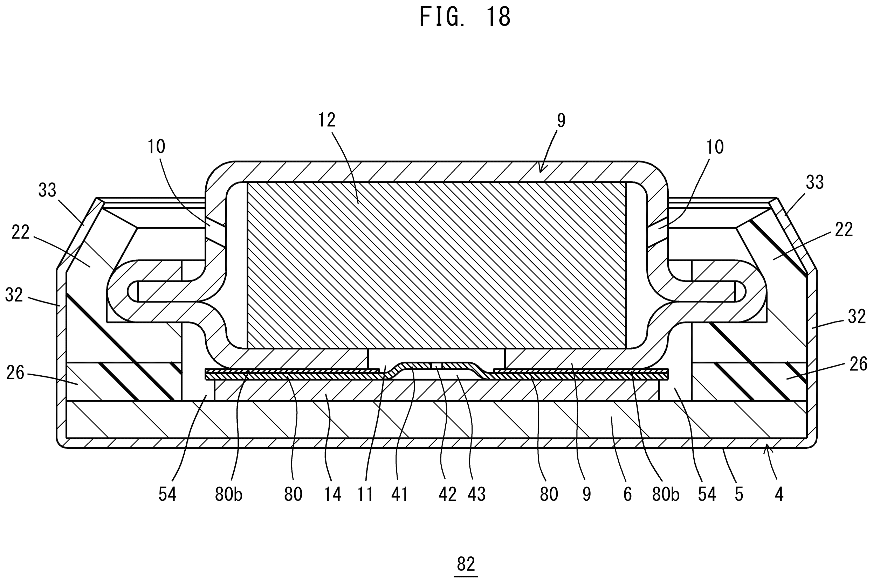

In the gas sensor 82 of Fig. 18, an electrically conductive film 80b is provided on the top surface of the diffusion control plate 80 facing the sealing cap 9. The film 80b may be, for example, a vapor deposited In film, a plated In film, or an In foil, or another low-melting-point metal film made of Ga alloys, Sn alloys or the like. The melting point of In is approximately 160°C, and the melting point of low-melting-point metals is at least 120°C or higher and less than 200°C. The film thickness is, for example, 1 µm or more and 10 µm or less, and the film thickness is made larger than the surface unevenness of the diffusion control plate 80 and the sealing cap 9.

The film 80b has a relatively low melting point and is soft near room temperature and deforms under caulking pressure. Consequently, the interface between the diffusion control plate 80 and the bottom surface of the sealing cap 9 is filled with In, generating an airtight seal. If the melting point is higher than In's, caulking should be performed under heated conditions, e.g., at 80 to 150°C, to avoid thermally affecting the performance of the proton conductor gas sensor. The film 80b may be provided on the bottom surface of the sealing cap 9 instead of the diffusion control plate. The film 80b may be provided on the bottom surface of the sealing cap 9, not on the diffusion control plate.

Instead of the film 80b consisting of the low-melting-point metal, an electrically conductive carbon powder such as graphite or carbon black, or a metal powder such as silver or copper, both mixed with a resin binder, etc., can be used to form the film for sealing. A 3D printing, a spin coater, or a printer are adequate for producing the sealing film 80b. In this case, the plastically deformable film 80b deforms by the pressure during caulking and fills the unevenness between the diffusion control plate 80 and the sealing cap 9.

Fig. 19 shows the changes in CO sensitivity due to the heat resistance test and drop test in the second embodiment (Fig. 10), while Fig. 20 shows the changes in the best embodiment (Figs. 14 and 15). The test procedure was the same as that described in the gas sensor test, with two samples used in Fig. 19 and three samples in Fig. 20. In the best embodiment, the sensitivity changes due to the heat resistance test and drop test were further smaller than in the second embodiment. This indicates the presence of an uncontrolled gas flow path between the diffusion control plate and the bottom surface of the sealing cap. Furthermore, it indicates that heating and dropping caused slight misalignment between the diffusion control plate and the sealing cap, altering the air permeability and causing the changes in gas sensitivity. In Fig. 19 (the second embodiment), the change in sensitivity due to the heat resistance test was greater than in Fig. 8 (the first and basic embodiment). However, the cause of this is unknown.

### List of Symbols

- 2: Proton Conductor Gas Sensor
- 4: Metal Can
- 5: bottom Portion
- 6: Bottom Plate
- 7: Side Peripheral
- 8: Caulked Part
- 9: Sealing Cap
- 10: Opening (for outside air intake)
- 11: Opening (for supplying outside air)
- 12: Filter
- 14: MEA
- 15: Proton Conductor Membrane
- 16: Detection electrode
- 17: Counter Electrode
- 18, 19: Gas Diffusion Membrane
- 20: Diffusion Control Plate
- 21: Diffusion Control Hole
- 22: Gasket
- 23: Protrusion
- 24: Bottom Portion
- 26, 30: O-ring
- 32: Flange
- 36: Proton Conductor Gas Sensor
- 40: Diffusion Control Plate
- 41: Protrusion
- 42: Diffusion Control Hole
- 43: Gap
- 46: Assembly of Proton Conductor Membrane and a Pair of Electrodes
- 50, 60, 70, 80: Diffusion Control Plate
- 50b, 60b, 70b: Extended Part
- 50c: Insulating Film
- 52, 62, 72, 82: Proton Conductor Gas Sensor
- 54: Gap
- 70d: Outer Circumference
- 74: Insulating Film
- 80b: Electrically Conductive Film

## Claims

1. A proton conductor gas sensor (2,36,52,62,72,82) comprising:
a metal can (4) having a flat bottom portion (5) and a ring-shaped side portion (7);
an MEA (membrane electrode assembly) (14) having a proton conductor membrane (15) and positioned over the bottom portion (5) of the metal can (4);
a sealing cap (9) made of metal, having an internal space accommodating a filter (12) , a first opening (10) introducing outside air into the internal space, and a second opening (11) supplying the outside air processed by the filter (12) to the MEA (14), positioned on an opposite side of the bottom portion (5) of the metal can (4) from the MEA (14); and
a gasket (22) having a ring shape and positioned between the side portion (7) of the metal can (4) and the sealing cap (9),
wherein an edge (8) of the side portion (7) of the metal can (4) on an opposite side of the bottom portion (5) of the metal can (4) is bent towards the sealing cap (9) and presses the gasket (22), being **characterized**
**in that** the gasket (22) is made of synthetic resin; and
that the proton conductor gas sensor (2,36,52,62,72,82) further comprises a synthetic rubber member (26,30) having a ring shape, more easily deformable than the gasket (22) by pressure, airtightly positioned between a bottom portion (5) of the gasket (22) and the bottom portion (5) of the metal can (4), and surrounding the MEA (14).

2. The proton conductor gas sensor (2,36,52,62,72,82) according to claim 1, being **characterized in that** a bottom plate (6) having a circular disk-like shape, made of metal, and having a thickness larger than that of the bottom portion (5) of the metal can (4), is positioned on the bottom portion (5) of the metal can (4), and
that the MEA (14) and the synthetic rubber member (26,30) are positioned on the bottom plate (6).

3. The proton conductor gas sensor (2,36,52,62,72,82) according to claim 1, being **characterized in that** the gasket (22) comprises at least one member of high-density polyethylene, nylon, and PTFE (polytetarafluoro-ethylene), and
that the synthetic rubber member (26,30) comprises at least one member of NBR rubber, FKM (fluoro-elastomer), PTFE rubber (polytetrafluoro-ethylene rubber), and nitrile rubber.

4. The proton conductor gas sensor (2,36,52,62,72,82) according to claim 3, being **characterized in that** the synthetic rubber member (26,30) is an O-ring made of synthetic rubber.

5. The proton conductor gas sensor (2,36,52,62,72,82) according to claim 1, being **characterized in that** the synthetic rubber member (26,30) is configured to reduce the increase in gas sensitivity due to drop impact test.

6. The proton conductor gas sensor (36,52,62,72,82) according to claim 1, being **characterized in that** a diffusion control plate (40,50,60,70,80) having a diffusion control hole (42) for supplying the outside air from the second opening (11) to the MEA (14) is provided and positioned between the MEA (14) and the sealing cap (9),
that the diffusion control hole (42) is smaller in size than the second opening (11),
that the diffusion control plate (40,50,60,70,80) has a protrusion (41) around the diffusion control hole (42) and protruding to the opposite side of the MEA (14),
and
that a gap (43) is provided between the protrusion (41) and the MEA (14).

7. The proton conductor gas sensor (52,62,72) according to claim 1, being **characterized in that** a diffusion control plate (50,60,70), having a diffusion control hole (42) for supplying the outside air from the second opening (11) to the MEA (14), is provided and positioned between the MEA (14) and the sealing cap (9), that the diffusion control hole (42) is smaller in size than the second opening (11), and
that an outer circumference of the diffusion control plate (50,60,70) is positioned between the gasket (22) and the synthetic rubber member (26,30).

8. The proton conductor gas sensor (52,62,72) according to claim 7, being **characterized in that** the outer circumference of the diffusion control plate (50,60,70) is entirely positioned between the gasket (22) and the synthetic rubber member (26,30).

9. The proton conductor gas sensor (82) according to claim 1, being **characterized in that** a diffusion control plate (80), having a diffusion control hole (42) for supplying the outside air from the second opening (11) to the MEA (14), is provided and positioned between the MEA (14) and the sealing cap (9),
that the diffusion control hole (42) is smaller in size than the second opening (11); and
that a film (80b) of an electrically conductive material, sealing airtightly an interface between the diffusion control plate (80) and the sealing cap (9), is provided on a top surface of the diffusion control plate (80) or on a bottom surface of the sealing cap (9).
